# EUROPEAN PATENT SPECIFICATION

(11) **EP 2 155 271 B1**
(45) Date of publication and mention of the grant of the patent: **08.03.2017**
(21) Application number: 08755679.1
(22) Date of filing: 16.05.2008
(51) Int. Cl.: A61L 12/14

(54) **PHOSPHOLIPID COMPOSITIONS FOR CONTACT LENS CARE AND PRESERVATION OF PHARMACEUTICAL COMPOSITIONS**
PHOSPHOLIPIDZUSAMMENSETZUNGEN ZUR KONTAKTLINSENPFLEGE UND KONSERVIERUNG PHARMAZEUTISCHER ZUSAMMENSETZUNGEN
COMPOSITIONS DE PHOSPHOLIPIDE POUR ENTRETIEN DE LENTILLE DE CONTACT ET PRESERVATION DE COMPOSITIONS PHARMACEUTIQUES

(30) Priority: 18.05.2007 US 938939 P
(43) Date of publication of application: 24.02.2010
(73) Proprietor: NOVARTIS AG, 4056 Basel (CH)
(72) Inventor: GHOSH, Malay, Fort Worth, Texas 76109 (US); CHOWHAN, Masood, A., Arlington, Texas 76016 (US); SCHNEIDER, L. Wayne., Crowley, Texas 76036 (US)
(74) Representative: Teipel, Stephan
(86) International application number: PCT/US2008/063877
(87) International publication number: WO 2008/144494

(56) References cited:
- EP-A- 0 427 548
- WO-A-94/20598
- WO-A-95/01414
- WO-A-95/27515
- WO-A-2008/065451
- US-A- 6 120 758

## Description

### BACKGROUND OF THE INVENTION

The present invention is directed to pharmaceutical compositions having antimicrobial activity, solutions for treating contact lenses having antimicrobial activity, and to the use of phospholipids in such compositions and solutions. More specifically, the invention is directed to use of phospholipid compounds of formula (I) in the preservation of various types of pharmaceutical compositions from microbial contamination, particularly ophthalmic, otic and nasal pharmaceutical compositions. Additionally, the present invention is directed to methods for disinfecting contact lenses.

Many pharmaceutical compositions are required to be sterile, i.e., free of bacteria, fungi and other pathogenic microorganisms. Examples of such compositions include:
solutions and suspensions that are injected into the bodies of humans or other mammals; creams, lotions, solutions or other preparations that are topically applied to wounds, abrasions, bums, rashes, surgical incisions, or other conditions where the skin is not intact; and various types of compositions that are applied either directly to the eye (e.g., artificial tears, irrigating solutions, and drug products), or are applied to devices that will come into contact with the eye (e.g., contact lenses).

The foregoing types of compositions can be manufactured under sterile conditions via procedures that are well known to those skilled in the art. However, once the packaging for a product is opened, such that the composition contained therein is exposed to the atmosphere and other sources of potential microbial contamination (e.g., the hands of a human patient), the sterility of the product may be compromised. Such products are typically utilized multiple times by the patient, and are therefore frequently referred to as being of a "multi-dose" nature.

There is a need for an improved means of preserving pharmaceutical compositions from microbial contamination. This need is particularly prevalent in the fields of ophthalmic, otic and nasal compositions, wherein the antimicrobial agents utilized to preserve the compositions must be effective in preventing microbial contamination of the compositions at concentrations that are non-toxic to ophthalmic, otic and nasal tissues.

Prior multi-dose ophthalmic compositions have generally contained one or more antimicrobial preservatives in order to prevent the proliferation of bacteria, fungi and other microbes. Such compositions may come into contact with the cornea either directly or indirectly. The cornea is particularly sensitive to exogenous chemical agents. Consequently, in order to minimize the potential for harmful effects on the cornea, it is preferable to use anti-microbial preservatives that are relatively non-toxic to the cornea, and to use such preservatives at the lowest possible concentrations, i.e., the minimum amounts required in order to perform their anti-microbial functions.

Balancing the anti-microbial efficacy and potential toxicological effects of anti-microbial preservatives is sometimes difficult to achieve. More specifically, the concentration of an antimicrobial agent necessary for the preservation of ophthalmic formulations from microbial contamination may create the potential for toxicological effects on the cornea and/or other ophthalmic tissues. Using lower concentrations of the anti-microbial agents generally helps to reduce the potential for such toxicological effects, but the lower concentrations may be insufficient to achieve the required level of biocidal efficacy, i.e., antimicrobial preservation.

The use of an inadequate level of antimicrobial preservation may create the potential for microbial contamination of the compositions and ophthalmic infections resulting from such contaminations. This is also a serious problem, since ophthalmic infections involving Pseudomonas aeruginosa or other virulent microorganisms can lead to loss of visual function or even loss of the eye.

Thus, there is a need for a means of enhancing the activity of anti-microbial agents so that very low concentrations of the agents can be utilized without increasing the potential for toxicological effects or subjecting patients to unacceptable risks of microbial contamination and resulting ophthalmic infections.

Phospholipids are phosphorus-containing lipids composed primarily of fatty acid chains, a phosphate group and a nitrogenous base. Of the lipids present in most cellular membranes, it is the phospholipids that provide the structural components for the membrane. Phospholipid molecules are amphiphilic and zwitterionic in nature, wherein the hydrophobic properties of such molecules are ascribed to the presence of long hydrocarbon chains and the hydrophilic properties of the molecule are derived from the charges carried by the phosphate and amino groups. In a typical phospholipid, the solubility properties of the molecule are dependent upon the length of the hydrocarbon chain(s) and the ionic functional groups.

Phospholipids are used extensively in various areas of biological science, such as in the cosmetic industry, pharmaceutical industry and in the preparation of other commercial products. In particular, phospholipids (synthetic or natural) are used in the pharmaceutical industry to prepare liposome-based formulations. Currently, over seven liposome products are available on the market in various disciplines, and several are undergoing development. In all cases, the active drugs are encapsulated in liposome vesicles, are available in a sterile unit dosage form, and no additional preservative ingredients are utilized.

Additional uses for phospholipids in the medical and pharmaceutical arts are described in U.S. Patent No. 5,286,719 (Fost et al.), which discloses a method for protecting substrates subject to contact by infectious viral organisms by treating such substrates with virucidally effective amounts of a composition containing a synthetic phospholipid as defined therein, and U.S. Patent Nos. 5,650,402 (Fost et al.) and 5,648,348 (Fost et al.), which disclose antimicrobial phospholipids that exhibit broad spectrum antibacterial and antifungal activity that are suitable for use as preservative and/or disinfectant agents in personal care and household products. However, these references do not disclose the use of phospholipids alone to preserve a pharmaceutical composition without the need for conventional preservative ingredients, such as benzalkonium chloride.

U.S. Patent No. 6,120,758 (Siddiqui et al.) discloses a preservative system for topically applied cosmetic, skin care, and pharmaceutical products (e.g., dermatologic, otic and ophthalmic preparations), including one or more of benzyl alcohols, disodium EDTA, and a para-hydroxybenzoic acid, in an effective antimicrobial amount, combined with one or more enhancers selected from the group consisting of sorbic acid, salts of sorbic acid, benzoic acid, salts of benzoic acid and certain phospholipids. However, it does not disclose that the phospholipids alone may be utilized to preserve a pharmaceutical drug composition without the need for conventional preservative ingredients, such as, benzalkonium chloride, nor does it disclose the use of the phospholipid compositions for contact lens care.

WO 95/27515 discloses compositions comprising water, hydrogen peroxide in an amount effective to disinfect contact lens, and a phosphate quaternary component in an amount effective in preserving said composition.

Contact lenses are exposed to a broad spectrum of microbes during normal wear and become soiled relatively quickly. Routine cleaning and disinfecting of the lenses are therefore required. Although the frequency of cleaning and disinfecting may vary somewhat among different types of lenses and lens care regimens, daily cleaning and disinfecting is normally required. Failure to clean and disinfect the lens properly can lead to a multitude of problems ranging from mere discomfort when the lenses are being worn to serious ocular infections. Ocular infections caused by particularly virulent microbes, such as *Pseudomonas aeruginosa,* can lead to loss of the infected eye(s) if left untreated or if allowed to reach an advanced stage before treatment is initiated. It is therefore extremely important that patients disinfect their contact lenses in accordance with the regimen prescribed by their optometrist or ophthalmologist.

Unfortunately, patients frequently fail to follow the prescribed regimens. Many patients find regimens to be difficult to understand and/or complicated, and as a result do not comply with one or more aspects of the regimen. Other patients may have a negative experience with the regimen, such as ocular discomfort attributable to the disinfecting agent, and as a result do not routinely disinfect their lenses or otherwise stray from the prescribed regimen. In either case, the risk of ocular infections is exacerbated.

Despite the availability of various types of contact lens disinfecting systems, such as heat, hydrogen peroxide, and other chemical agents, there continues to be a need for improved systems which: 1) are simple to use, 2) have potent antimicrobial activity, and 3) are nontoxic (i.e., do not cause ocular irritation even if the system were to bind to the lens material). There is also a need for chemical disinfecting agents that retain their antimicrobial activity in the presence of salts (e.g., sodium chloride) and other components of compositions utilized to treat contact lenses.

The present invention is directed to satisfying the above-cited needs.

### SUMMARY OF THE INVENTION

The present invention is based on the use of synthetic phospholipid compounds of formula (I) to enhance the antimicrobial activity of pharmaceutical compositions and to preserve pharmaceutical compositions from contamination by microorganisms. The invention is particularly directed to ophthalmic, otic and nasal compositions of this kind, but is also applicable to various other types of pharmaceutical compositions. The invention is further directed to contact lens care solutions containing one or more synthetic phospholipids of formula (I) and to methods for disinfecting contact lenses with such solutions.

The synthetic phospholipids utilized in the present invention have unique molecular arrangements wherein a phosphate group is linked to a quaternary ammonium functionality via a substituted-propenyl group, and the quaternary ammonium functionality is further linked to at least one long hydrocarbon chain. Such molecular arrangements are what make the phospholipids of formula (I) highly water soluble. In particular, the length of the hydrocarbon chain and the ionic functional groups are important factors to consider for maintaining solubility and efficacy of the molecules for the uses described herein.

The presence of quaternary ammonium functional groups is also a feature of known antimicrobial preservatives, such as benzalkonium chloride, and polyquaternium-1. These functional groups bear a positive charge and as a result tend to interact with negatively charged molecules or ions in solution. Such interactions may adversely affect the ability of the quaternary ammonium compounds to interact with negatively charged sites on the cell walls of microbes, thereby compromising the antimicrobial activity of the compounds.

The present invention is based in-part on the finding that the synthetic phospholipids of formula (I) are potent antimicrobial agents and capable of preserving pharmaceutical compositions from microbial contamination without the use of conventional antimicrobial agents, such as benzalkonium chloride or polyquaternium-1, but are particularly susceptible to deactivation in the presence of negatively and positively charged molecules or ions, e.g., sodium and chloride from sodium chloride. The positively charged sodium ions from sodium chloride compete with the positive charge of the preservative to bind on the negative sites of a microorganism, while the presence of additional negatively charged chloride ions increases the probability of interaction with positively charged sites on the preservative.

The present inventors have found that this property of the synthetic phospholipids of formula (I) makes these compounds particularly useful as antimicrobial preservatives for ophthalmic pharmaceutical compositions, because the anions found in the lacrimal fluid of the eye, i.e., tear fluid, interact with the compounds of formula (I) thereby neutralizing the compounds. This neutralization effectively reduces or prevents the ocular irritation that has been frequently associated with the use of conventional quaternary ammonium antimicrobial preservatives, particulary benzalkonium chloride. Thus, the synthetic phospholipids of formula (I) have been found to be very useful to preserve pharmaceutical compositions from microbial contamination during storage, and have the additional advantage of being very gentle when applied to the human eye, due to the above-discussed neutralization effects.

The compounds of formula (I) may be utilized as antimicrobial preservatives for the compositions of the present invention in place of conventional, antimicrobial agents known to those skilled in the art, for example, benzalkonium chloride. More specifically, the pharmaceutical compositions of the present invention are preserved without the need for conventional antimicrobial preservative agents, such as benzalkonium chloride, benzalkonium bromide, polyquaternium-1, chlorhexidine, chlorobutanol, cetylpyridinium chloride, parabens, thimerosal, chlorine dioxide and N,N-dichlorotaurine.

### DETAILED DESCRIPTION OF THE INVENTION

The present invention is directed to sterile compositions comprising preservative effective amount of a synthetic phospholipid of the formula: 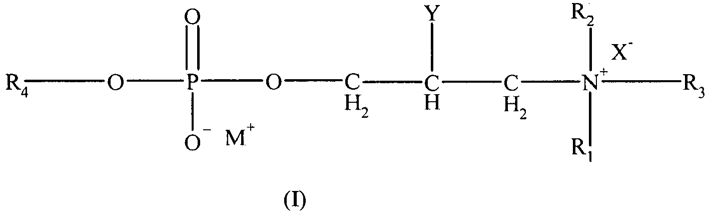 wherein:
R₁ and R₃ are (C₁-C₆)-alkyl;
R₂ is selected from the group consisting of hydrogen and (C₁-C₁₆)-alkyl optionally substituted by NHC(=O)-(CH₂)₁₀CH₃ or NHC(=O)-(CH₂)₁₂CH₃;
R₄ is selected from the group consisting of hydrogen and CH₂CH(Y)CH₂N⁺R₁R₂R₃X⁻, wherein R₁, R₂, and R₃, are as defined above;
X is halo;
Y is selected from the group consisting of OH, O-(C₁-C₁₀)-alkyl and O-(C₁-C₁₀)-alkenyl; and
M is selected from the group consisting of sodium and potassium;
a physiologically suitable buffer, and a pharmaceutically acceptable vehicle therefor, wherein the composition has an ionic strength of not greater than 0.12, wherein the amount of the compound of formula (I) is 0.001% (w/v) to 0.005% (w/v), and which composition does not contain other antimicrobial agents.

In the foregoing definitions of R₁, R₂, R₃, R₄, X, Y and M substituents, and throughout, the following terms unless otherwise indicated, shall be understood to have the following meanings:
The term "alkenyl" includes straight or branched chain hydrocarbon groups having 1 to 30 carbon atoms with at least one carbon-carbon double bond, the chain being optionally interrupted by one or more heteroatoms. The chain hydrogens may be substituted with other groups, such as, halo, -CF₃, -NO₂, -NH₂, -CN, -OCH₃, -C₆H₅, -O-C₆H₅O-alkyl, -O-C₆H₅O-alkenyl, p-NHC(=O)-C₆H₅-NHC(=O)-CH₃, -CH=NH, -NHC(=O)-Ph and -SH. Preferred straight or branched alkenyl groups include allyl, ethenyl, propenyl, butenyl pentenyl, hexenyl, heptenyl, octenyl, nonenyl, decenyl, undecenyl, dodecenyl, tridecenyl, tetradecenyl, pentadecenyl or hexadecenyl.

The term "alkyl" includes straight or branched chain aliphatic hydrocarbon groups that are saturated and have 1 to 30 carbon atoms. The alkyl groups may be interrupted by one or more heteroatoms, such as oxygen, nitrogen, or sulfur, and may be substituted with other groups, such as, halo, -CF₃, -NO₂, -NH₂, -CN, -OCH₃, -C₆H₅, -O-C₆H₅O-alkyl, -O-C₆H₅O-alkenyl, p-NHC(=O)-C₆H₅-NHC(=O)-CH₃, -CH=NH, -NHC(=O)-Ph and -SH. Preferred straight or branched alkyl groups include methyl, ethyl, propyl, isopropyl, butyl, t-butyl, sec-butyl, pentyl, hexyl, heptyl, octyl, nonyl, decyl, undecyl and dodecyl, tridecyl, tetradecyl, pentadecyl or hexadecyl.

The term "halo" means an element of the halogen family. Preferred halo moieties include fluorine, chlorine, bromine or iodine.

The unique molecular arrangement of the synthetic phospholipids (i.e., wherein a phosphate group is linked to a quaternary ammonium functionality via a substituted-propenyl group, and the quaternary ammonium functionality is further linked to at least one long hydrocarbon chain) are what make them highly water soluble. In particular, the length of the hydrocarbon chain and the ionic functional groups are important factors to consider for maintaining solubility and efficacy of the molecules for the uses described herein.

The preferred compounds of formula (I) are those wherein R₁ and R₃ are methyl; R₂ is selected from the group consisting of (CH₂)₁₁CH₃, (CH₂)₃-NHC(=O)-(CH₂)₁₀CH₃ and (CH₂)₃-NHC(=O)-(CH₂)₁₂CH₃; R₄ is CH₂CH(Y)CH₂N⁺R₁R₂R₃X⁻, wherein R₁, R₂, and R₃, are as defined above; X is chloro; Y is OH; and M is sodium. The most preferred compounds are identified in the following table:

| **SUBSTITUENT** | **COMPOUND NO. 1 (PHOSPHOLIPID CDM)** | **COMPOUND NO. 2 (PHOSPHOLIPID PTC)** | **COMPOUND NO. 3 (PHOSPHOLIPID PTM)** |
|---|---|---|---|
| **R₁** | -CH₃ | -CH₃ | -CH₃ |
| **R₂** | -(CH₂)₁₁CH₃ | -(CH₂)₃-NHC(=O)-(CH₂)₁₀CH₃ | -(CH₂)₃-NHC(=O)-(CH₂)₁₂CH₃ |
| **R₃** | -CH₃ | -CH₃ | -CH₃ |
| **R₄** | 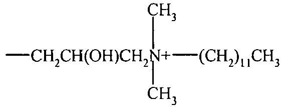 | 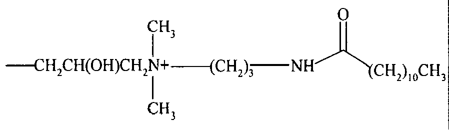 | 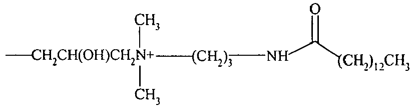 |
| **X⁻** | Cl⁻ | Cl⁻ | Cl⁻ |
| **Y** | -OH | -OH | -OH |
| **M**⁺ | Na⁺ | Na⁺ | Na⁺ |

Compound Number 1 is the most preferred compound of formula (I).

The compounds of formula (I) can be synthesized in accordance with known procedures (see for example, U.S. Patent Nos. 5,286,719; 5,648,348 and 5,650,402) and/or purchased from commercial sources, such as Uniquema (Cowick Hall, Snaith, Goole East Yorkshire, DN149AA).

As described above, the synthetic phospholipids of formula (I) have unique molecular arrangements and physical properties relative to other phospholipids that make them highly water soluble and particularly efficacious for the uses described herein. The affinity of the compounds for ionic interactions is one such property.

The ionic strength of the compositions of the present invention has been found to be an important factor for achieving preservation or disinfection with the compound of formula (I). More specifically, the compositions lose antimicrobial activity when the concentration of anionic agents in the compositions is increased. Consequently, it is important to limit the amount of ionic solutes present in the composition of the present invention, so as to avoid a loss of antimicrobial activity that adversely affects the ability of the compound of formula (I) to preserve the compositions from microbial contaimination and/or to disinfect contact lenses. This principle is further illustrated in Example 2 below (see Formulations I through K). The use of solutions having low ionic strengths, i.e., low concentrations of ionic solutes such as sodium chloride, is therefore preferred. Examples of ionic solutes include potassium chloride, magnesium chloride and calcium chloride. As utilized herein, the term "ionic strength" means a measure of the average electrostatic interactions among ions in an electrolyte; it is equal to one-half the sum of the terms obtained by multiplying the molality of each ion by its valence squared.

The compounds of formula (I) may also be included in various types of pharmaceutical compositions as preservatives, so as to prevent microbial contamination of the compositions. The types of compositions which may be preserved by the compounds of formula (I) include: (a) ophthalmic pharmaceutical compositions, such as topical compositions used in the treatment of glaucoma, infections, various retinal diseases, allergies or inflammation; (b) otic pharmaceutical compositions, such as topical compositions used in the treatment of bacterial infections or inflammation of the ear; (c) nasal pharmaceutical compositions, such as topical compositions used in the treatment of rhinitis; (d) compositions for treating contact lenses, such as cleaning products and products for enhancing the ocular comfort of patients wearing contact lenses; (e) other types of ophthalmic compositions, such as ocular lubricating products, artificial tears, astringents, and so on; (f) dermatological compositions, such as antiinflammatory compositions, as well as shampoos and other cosmetic compositions; and (f) various other types of pharmaceutical compositions.

The present invention is not limited with respect to the types of pharmaceutical compositions containing compound(s) of formula (I) as preservatives, but the compounds are particularly useful in preserving ophthalmic, otic and nasal compositions from microbial contamination. The compounds are particularly useful in these types of compositions due to the ability of the compounds to exhibit a preservative effect at very low concentrations, without adversely affecting ophthalmic, otic and nasal tissues. In particular, when compound(s) of formula (I) are applied to the eye, they have significantly less effect on ocular tissues due to ionic neutralization and/or dilution effects in the presence of lacrimal fluid, i.e., tears.

The compositions of the present invention may be formulated as aqueous solutions. Additionally, the compositions may be formulated as suspensions, gels, emulsions and other dosage forms known to those skilled in the art.

The ophthalmic, otic and nasal compositions of the present invention will be formulated so as to be compatible with the eye, ear, nose and/or contact lenses to be treated with the compositions. As will be appreciated by those skilled in the art, ophthalmic compositions intended for direct application to the eye will be formulated so as to have a pH and tonicity, i.e., osmolality, that are compatible with the eye. This will normally require a buffer to maintain the pH of the composition at or near physiologic pH (i.e., 7.4) and may require a tonicity-adjusting agent (e.g., NaCl) to bring the osmolality of the composition to a level that ranges from slightly hypotonic to isotonic, relative to human tears.

The ophthalmic compositions of the present invention will contain a preservative effective amount of one or more synthetic phospholipids of formula (I) and an ophthalmically acceptable vehicle. As utilized herein, the term "opthalmically acceptable vehicle" means a pharmaceutical composition having physical properties (e.g., pH and/or osmolality) that are physiologically compatible with ophthalmic tissues.

A preferred range of osmolality for the ophthalmic compositions of the present invention is 150 to 350 milliOsmoles per kilogram (mOsm/kg). A range of 200 to 300 mOsm/kg is particularly preferred and an osmolality of about 275 mOsm/kg is most preferred. The pH for the ophthalmic compositions of the present invention range from about 4.5 to about 9.0.

The pharmaceutical compositions of the present invention may contain one or more active ingredients. As utilized herein, the term "active ingredient" means a compound that causes a physiological effect for a therapeutic purpose, e.g., a compound that lowers or controls intraocular pressure in the treatment of glaucoma, and is therefore functioning as a drug.

The compositions of the present invention will contain one or more synthetic phospholipids of formula (I). The concentrations of the compounds in the compositions will depend on the purpose of the use, e.g., preservation of pharmaceutical compositions, and the absence or inclusion of other antimicrobial agents. The concentrations determined to be necessary for the above-stated purposes can be functionally described as "an amount effective to preserve" or variations thereof as described below. The term "effective to preserve" means an amount of an antimicrobial agent effective in producing the desired effect of preserving the compositions described herein from microbial contamination, preferably an amount which, either singly or in combination with one or more additional antimicrobial agents, is sufficient to satisfy the preservative efficacy requirements of at least the United States Pharmacopoeia ("USP"), 29th Revision, The National Formulary, United States Pharmacopoeial Convention, Inc., Rockville, MD 2256-2259. The concentrations used are in the range of from 0.001 to 0.005 weight/volume percent (w/v %).

The active ingredient or ingredients that can be included in the compositions of the present invention include, but are not limited to, ophthalmic, otic or nasal agents that can be topically applied. For example, such ophthalmic agents include (but are not limited to): anti-glaucoma agents, such as beta-blockers (e.g., betaxolol and timolol), muscarinics (e.g., pilocarpine), prostaglandins, carbonic anhydrase inhibitors (e.g., acetazolamide, methazolamide and ethoxzolamide), dopaminergic agonists and antagonists, and alpha adrenergic receptor agonists, such as para-amino clonidine (also known as apraclonidine) and brimonidine; anti-infectives, such as ciprofloxacin; non-steroidal and steroidal anti-inflammatories, such as suprofen, ketorolac, dexamethasone, rimexolone and tetrahydrocortisol; proteins; growth factors, such as EGF; and anti-allergic agents, such as cromolyn sodium, emedastine and olopatadine. Other nonlimiting examples of therapeutic agents that may be used include but not limited to anticholinergic, sympathomimetic agents, antiangiogenic agents, anti vascular permeability agents, anaesthetics, analgesiscs, protease inhibitors, cell transport/mobility impending agents, anti-cytomegalovirus agents, immunological response modifiers, antineoplastics agents. Compositions of the present invention may also include combinations of active ingredients. Most preferred are topically administrable ophthalmic compositions.

As will be appreciated by those skilled in the art, the compositions of the present invention may contain a wide variety of ingredients, such as tonicity agents (e.g., sodium chloride, propylene glycol, mannitol), surfactants (e.g., polysorbate, cremophore, and polyoxyethylene/polyoxypropylene copolymers), viscosity adjusting agents (e.g., hydroxypropyl methyl cellulose, other cellulose derivatives, gums and derivatives of gums), buffering agents (e.g., borates, citrates, phosphates, carbonates) comfort-enhancing agents (e.g., guar gum, xanthan gum and polyvinyl pyrrolidone when appropriate and applicable), solubilizing aids, pH adjusting agents, antioxidants, or complexing agents (e.g., (ethylenedinitrilo)-tetraacetic acid disodium salt, also referred to as disodium EDTA, nonnyl ethylenediaminetriacetic acid) and stabilizing agents.. The ability of the compositions of formula (I) to retain their antimicrobial activity in the presence of such agents is a significant advantage of the present invention.

The formulation of compositions for treating contact lenses (e.g., disinfecting and/or cleaning) will involve considerations similar to those described above for other types of ophthalmic compositions, as well as considerations relating to the physical effect of the compositions on contact lens materials and the potential for binding or absorption of the components of the composition by the lens. The contact lens disinfecting compositions of the present invention will preferably be formulated as aqueous solutions, as well as suspensions, gels, emulsions and so on. The compositions may contain a variety of tonicity agents, surfactants, viscosity adjusting agents and buffering agents, as described above.

The above-described compositions may be used to disinfect contact lenses in accordance with processes known to those skilled in the art. More specifically, the lenses will first be removed from the eyes of the patients, and then will be immersed in the compositions for a time sufficient to disinfect the lenses. This immersion will typically be accomplished by means of soaking the lenses in a solution for a period of time ranging from a few hours to overnight, i.e., four to eight hours. The lenses will then be rinsed and placed in the eye. Prior to immersion in the disinfecting compositions, the lenses will preferably also be cleaned and rinsed.

The compositions and methods of the present invention may be used in conjunction with various types of contact lenses, including both lenses generally classified as "hard" and lenses generally classified as "soft", as well as rigid and soft gas permeable lenses. Such suitable lenses may include silicone and fluorine containing lenses as well as both hydrogel and non-hydrogel lenses. Furthermore, compositions of the present invention are not expected to discolor colored contact lenses. Compositions of the present invention comprise phospholipid compound(s) of formula (I) in an effective amount either alone or in combination with other antimicrobial agents in a physiologically suitable buffer. Illustrative examples of a disinfecting solution, a comfort drop solution for a contact lens user and a lubricant eye drop are provided in Examples and Ref. Examples 5-9 below.

As described above, the amount of each compound used will depend on the purpose of the use, e.g., disinfection of contact lenses, and the absence or inclusion of other antimicrobial agents. The concentrations determined to be necessary for the above-stated purposes can be functionally described as "an amount effective to disinfect" or variations thereof as described below. The term "effective to disinfect" means an amount of antimicrobial agent effective in producing the desired effect of disinfecting contact lenses by substantially reducing the number of viable microorganisms present on the lenses, preferably an amount which is sufficient to satisfy the disinfection requirements according to FDA Premarket Notification (510k) Guidance Document for Contact Lens Care Products (1997) and ISO/FDIS 14729: Ophthalmic optics-Contact lens care products-Microbiological requirements and test methods for products and regimens for hygienic management of contact lenses (2001). The concentrations used are in the range of from 0.001 to 0.005 w/v %.

The following examples are provided to further illustrate the use of the compounds of formula (I) in compositions of the present invention and to demonstrate the antimicrobial activity of the compounds.

### EXAMPLE 1

The following formulation represents an example of a preserved ophthalmic formulation of the present invention. In this formulation, the phospholipid compound of formula (I) functions to preserve the formulation from microbial contamination during storage.

**Composition of a Preserved Ophthalmic Formulation**

| Ingredient | Concentration (% w/v) |
|---|---|
| Olopatadine Hydrochloride | 0.05-0.25 |
| Phospholipid of Formula (I) | 0.001-0.005 |
| Disodium EDTA | 0-0.05 |
| Boric acid | 0-2 |
| Propylene glycol | 0-2 |
| Sodium chloride | 0-0.9 |
| Hydrochloric acid | q.s. to pH |
| Sodium hydroxide | q.s. to pH |
| Purified Water | q.s. to 100 |
| pH | q.s. to 6.0 - 8.0 |

Preparation of 0.1 % Preserved Ophthalmic Formulation (as reference Example): Olopatadine hydrochloride (0.111 g) and boric acid (1.0 g) were combined in purified water (-75 mL) and stirred for approximately 30 minutes. To this was added propylene glycol (0.3 g), and then sodium chloride (0.5 g). The mixture was stirred well to dissolve. To the mixture was added phospholipid CDM (1.0 g of 1% stock solution prepared in water). A sufficient amount of purified water was added to bring the formulation to ∼95 g. The pH was adjusted to ∼7.0, by the addition sodium hydroxide solution (1N) and the final batch amount was then adjusted to 100 g by adding purified water. The formulation was sterilized by filtering through a 0.22 micron membrane filter in a laminar flow hood.

### EXAMPLE 2

The antimicrobial activity of the formulations shown in Table 1 below, containing 0.0001-1 (w/v %) of a phospholipid identified above as Compound No. 1 (Phospholipid CDM), Compound No. 2 (Phospholipid PTC) or Compound No. 3 (Phospholipid PTM), were evaluated relative to five microorganisms used in standard antimicrobial preservative efficacy testing. The evaluation was conducted by determining the extent to which the solution reduced an initial population of about 10⁶ cfu/mL microorganisms over time. The abbreviation "cfu" means colony forming units. The preservative efficacy results for the formulations are also presented in Table 1. It should be noted that Formulations A through V all have similar osmolalities of about 275 mOsm/kg while differing in relative ionic strength. Only Formulations B, F and N are according to the invention. The other formulations are described for reference purpose.

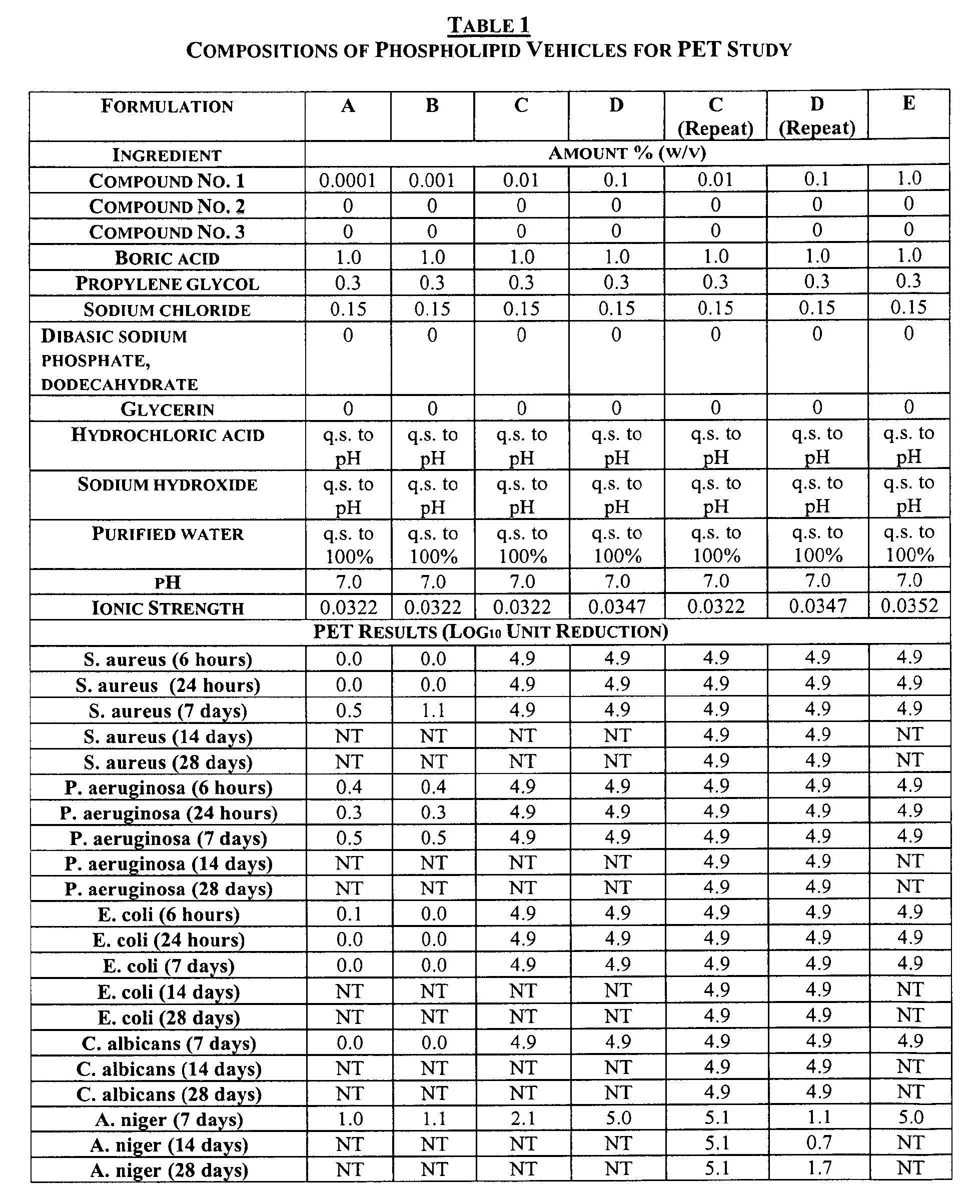

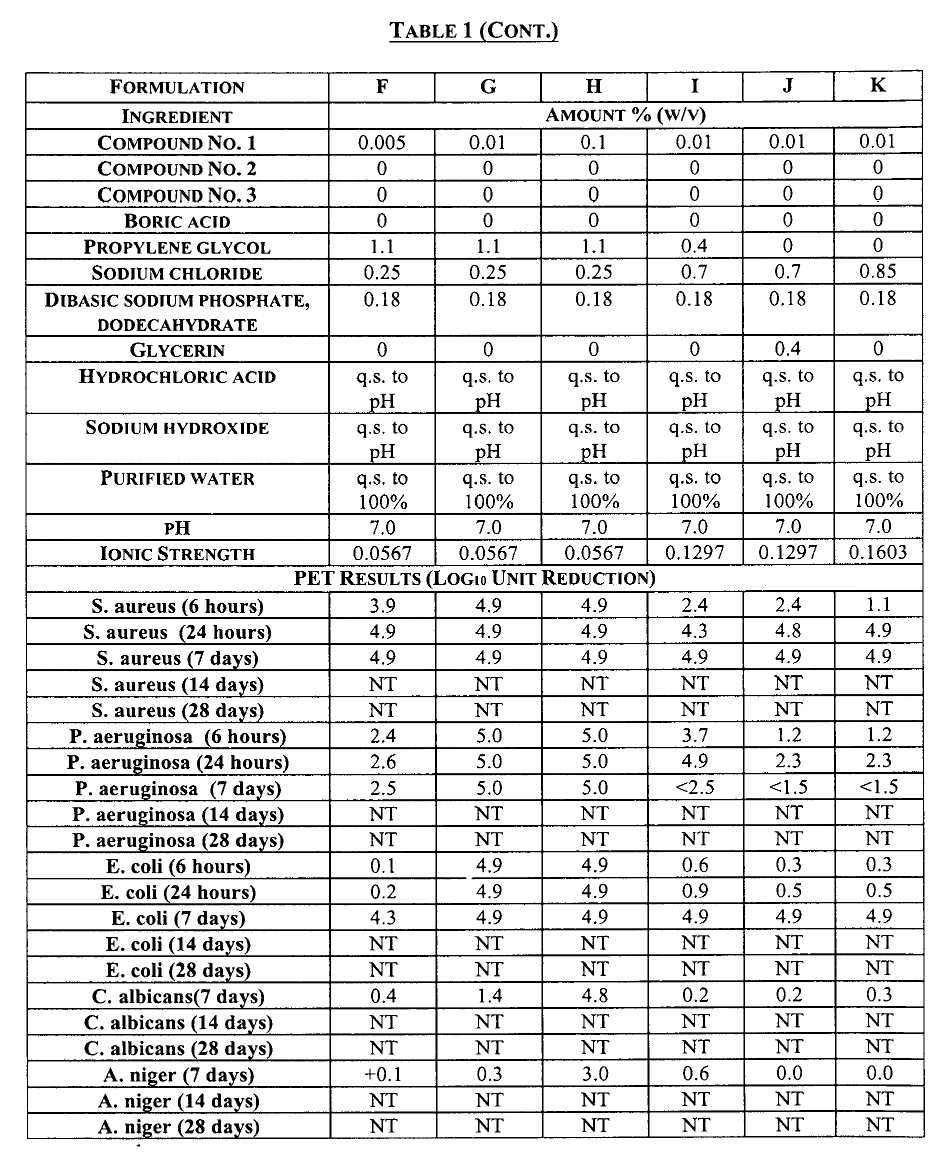

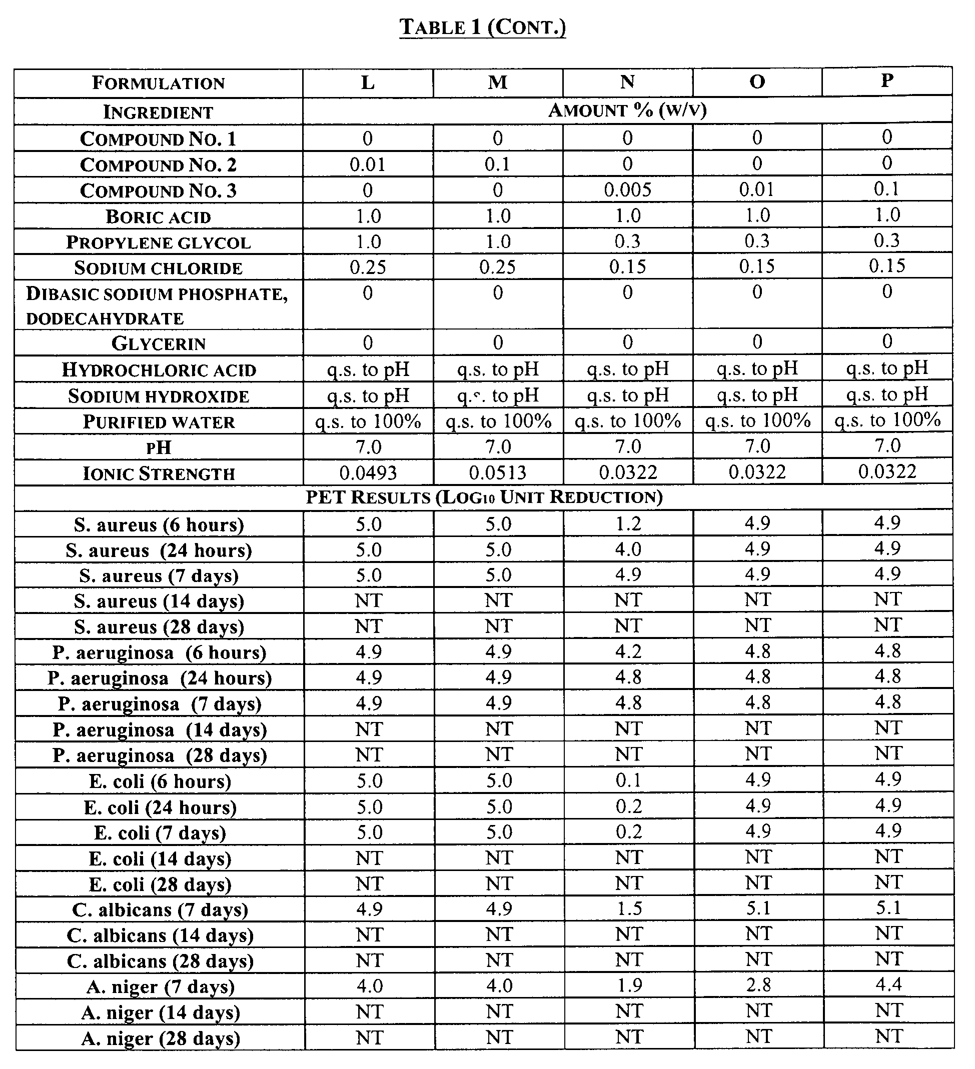

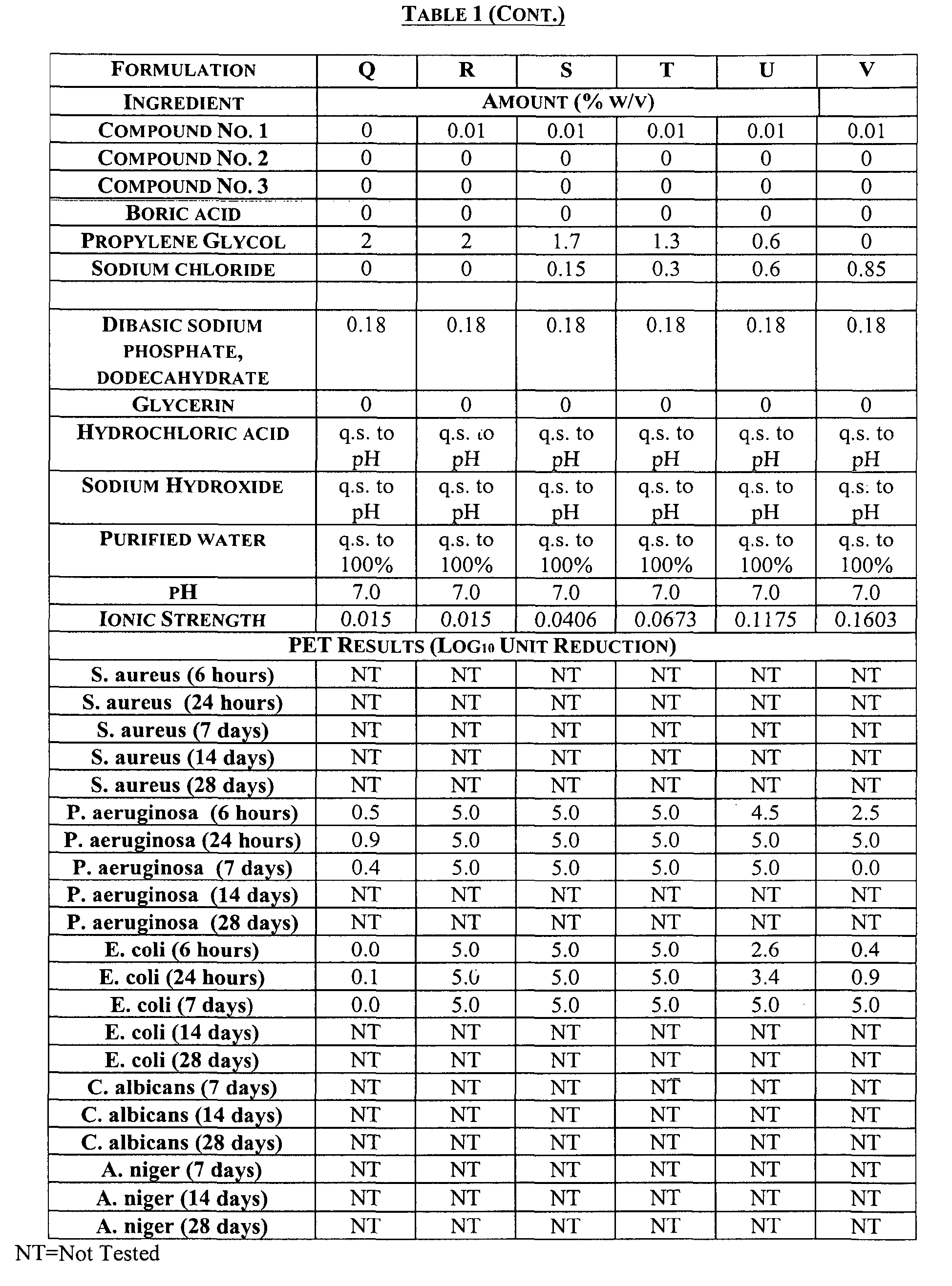

The results in Table 1 above demonstrate the potent antimicrobial activity of the synthetic phospholipids of formula (I). The results also demonstrate that the preservative efficacy depends in part upon both the concentration of the phospholipid present and the ionic strength of the composition. Formulations A through E together, Formulations F through H together and Formulations Q through V together each demonstrate that preservative efficacy is concentration dependent, i.e., preservative efficacy improves as phospholipid (Compound No. 1) concentration increases. Formulations I through K together demonstrate that preservative efficacy decreases as the ionic strength increases (relative to Formulations A through E and F through H) due to an increase in the amount of sodium chloride concentration, when the phospholipid (Compound No. 1) concentration is fixed at 0.01.

The following formulations 3 through 9 are aqueous, isotonic solutions. They can be prepared in a similar manner as the solution of Example 1 above.

### REF. EXAMPLE 3

**Ophthalmic Solution (preserved by benzalkonium chloride and phospholipid)**

| **Ingredient** | **Concentration (%, w/v)** |
|---|---|
| Olopatadine hydrochloride | 0.111 |
| Benzalkonium chloride | 0.005 |
| Phospholipid of Fomula (I) | 0.001-2 |
| Dibasic sodiumphosphate (anhydrous) | 0.5 |
| Sodium chloride | 0.6 |
| Hydrochloric acid | q.s. to pH |
| Sodium hydroxide | q.s. to pH |
| Purified Water | q.s. to 100 |
| pH | q.s. to pH 7.0 |

### EXAMPLE 4

**Otic or Nasal Formulation**

| **Ingredient** | **Concentration (%, w/v)** |
|---|---|
| Active Ingredient | 0.01-5 |
| Phospholipid of Formula (I) | 0.005 |
| Disodium EDTA | 0.001-0.05 |
| Dibasic SodiumPhosphate | 0-1 |
| Monobasic SodiumPhosphate | 0-1 |
| Sodium chloride | 0.5-0.9 |
| Hydrochloric acid | q.s. to pH |
| Sodium hydroxide | q.s. to pH |
| Purified Water | q.s. to 100 |
| pH | q.s. to 4.5 - 8.0 |

### EXAMPLE 5

**Disinfecting Solution**

| **Ingredient** | **Concentration (%, w/v)** |
|---|---|
| Phospholipid of Formula (I) | 0.001-0.005 |
| Disodium EDTA | 0.0001-0.05 |
| Boric acid | 0-2 |
| Propylene glycol | 0-1 |
| Sodium chloride | 0.5-0.9 |
| Hydrochloric acid | q.s. to pH |
| Sodium hydroxide | q.s. to pH |
| Purified Water | q.s. to 100 |
| pH | q.s. to 6.0-8.0 |

### EXAMPLE 6

**Comfort Drop Solution for Contact Lenses**

| **Ingredient** | **Concentration (%, w/v)** |
|---|---|
| Phospholipid of Formula (I) | 0.001-0.005 |
| Disodium EDTA | 0.0001-0.05 |
| Dibasic sodiumphosphate | 0-1 |
| Monobasic sodiumphosphate | 0-1 |
| Povidone | 0-2 |
| Sodium chloride | 0.5-0.9 |
| Hydrochloric acid | q.s. to pH |
| Sodium hydroxide | q.s. to pH |
| Purified Water | q.s. to 100 |
| pH | q.s. to 5.0 - 8.0 |

### EXAMPLE 7

**Lubricant Eye Drop**

| **Ingredient** | **Concentration (%, w/v)** |
|---|---|
| Phospholipid of Formula (1) | 0.001-0.005 |
| DisodiumEDTA | 0-0.05 |
| Dextran T 70 | 0-3 |
| Hydroxypropyl methylcellulose | 0-0.5 |
| I Sodium bicarbonate | 0-2 |
| Sodium chloride | 0.5-0.9 |
| Potassium chloride | 0.05-0.2 |
| Hydrochloric acid | q.s. to pH |
| Sodium hydroxide | q.s. to pH |
| Purified Water | q.s. to 100 |
| pH | q.s. to 6.5 -7.8 |

### REF. EXAMPLE 8

**Lubricant Eye Drop**

| **Ingredient** | **Concentration (%, w/v)** |
|---|---|
| HP-Guar | 0.16 |
| Phospholipid CDM | 0.01 |
| Boric Acid | 0.7 |
| Sorbitol | 1.4 |
| Polyethylene Glycol | 0.4 |
| Propylene Glycol | 0.3 |
| Potassium Chloride | 0.12 |
| Sodium Chloride | 0.1 |
| Calcium Chloride | 0.0053 |
| Magnesium Chloride | 0.0064 |
| Zinc Chloride | 0.00015 |
| AMP-95 | 0.6 |
| Sodium Hydroxide | q.s. to pH |
| Hydrochloric Acid | q.s. to pH |
| Purified Water | q.s. to 100 |
| pH | q.s. to pH 7.9 |

### REF. EXAMPLE 9

**Lubricant Eye Drop**

| **Ingredient** | **Concentration (%, w/v)** |
|---|---|
| HP 8A-Guar | 0.16-.019 |
| Phospholipid CDM | 0.01 |
| Boric Acid | 0.63 |
| Sorbitol | 1.26 |
| Polyethylene Glycol | 0.4 |
| Propylene Glycol | 0.3 |
| Potassium Chloride | 0.12 |
| Sodium Chloride | 0.1 |
| Calcium Chloride | 0.0053 |
| Magnesium Chloride | 0.0064 |
| Zinc Chloride | 0.00135 |
| AMP-95 | 0.513 |
| Sodium Hydroxide | q.s. to pH |
| Hydrochloric Acid | q.s. to pH |
| Purified Water | q.s. to 100 |
| pH | q.s. to pH 7.4-7.9 |

## Claims

1. A sterile pharmaceutical composition comprising a preservative effective amount of a compound of the following formula, 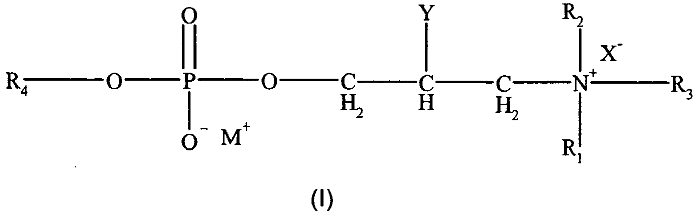 wherein:
R₁ and R₃ are (C₁-C₆)-alkyl;
R₂ is selected from the group consisting of hydrogen and (C₁-C₁₆)-alkyl optionally substituted by NHC(=O)-(CH₂)₁₀CH₃ or NHC(=OHCH₂)₁₂CH₃;
R₄ is selected from the group consisting of hydrogen and CH₂CH(Y)CH₂N⁺R₁R₂R₃X,
wherein R₁, R₂, and R₃, are as defined above;
X is halo;
Y is selected from the group consisting of OH, O-(C₁-C₁₀)-alkyl and O-(C₁-C₁₀)-alkenyl;
M is selected from the group consisting of sodium and potassium;
a physiologically suitable buffer, and a pharmaceutically acceptable vehicle therefor, wherein the composition has an ionic strength of not greater than 0.12, wherein the amount of the compound of formula (I) is 0.001% (w/v) to 0.005% (w/v), and which composition does not contain other antimicrobial agents.

2. The composition of claim 1, wherein:
R₁ and R₃ are methyl;
R₂ is selected from the group consisting of (CH₂)₁₁CH₃, (CH₂)₃-NHC(=O)-(CH₂)₁₀CH₃, and
(CH₂)₃-NHC(=O)-(CH₂)₁₂CH₃;
R₄ is CH₂CH(Y)CH₂N⁺R₁R₂R₃X⁻, wherein R_{1,} R₂, and R_{3,} are as defined above;
X is chloro;
Y is OH; and
M is sodium.

3. The composition of claim 2 wherein the compound of formula (I) is selected from the group consisting of Phospholipid CDM which is a compound of formula (I), wherein:
R₁ and R₃ are methyl;
R₂ is -(CH₂)₁₁CH₃;
R₄ is -CH₂CH(Y)CH₂N⁺R₁R₂R₃X⁻, wherein R₁, R₂, and R₃ are as defined above;
X is chloro;
Y is OH; and
M is sodium,
Phospholipid PTC which is a compound of formula (I), wherein:
R₁ and R₃ are methyl;
R₂ is -(CH₂)₃-NHC(=O)-(CH₂)₁₀CH₃;
R₄ is -CH₂CH(Y)CH₂N⁺R₁R₂R₃X⁻, wherein R₁, R₂, and R₃ are as defined above;
X is chloro;
Y is OH; and
M is sodium,
and Phospholipid PTM which is a compound of formula (I), wherein:
R₁ and R₃ are methyl;
R₂ is -(CH₂)₃-NHC(=O)-(CH₂)₁₂CH₃;
R₄ is CH₂CH(Y)CH₂N⁺R₁R₂R₃X⁻, wherein R₁, R₂, and R₃ are as defined above;
X is chloro;
Y is OH; and
M is sodium.

4. A method of preserving a pharmaceutical composition from microbial contamination which comprises adding to the composition a preservative effective amount of a compound of the following formula, 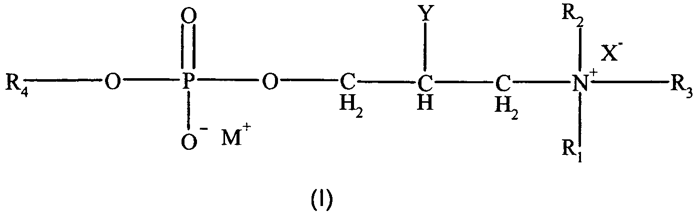 wherein:
R₁ and R₃ are (C₁-C₆)-alkyl;
R₂ is selected from the group consisting of hydrogen and (C₁-C₁₆)-alkyl optionally substituted by NHC(=O)-(CH₂)₁₀CH₃ or NHC(=O)-(CH₂)₁₂CH₃;
R₄ is selected from the group consisting of hydrogen and CH₂CH(Y)CH₂N⁺R₁R₂R₃X_{'} wherein R_{1,} R_{2,} and R_{3,} are as defined above;
X is halo;
Y is selected from the group consisting of OH, O-(C₁-C₁₀)-alkyl and O-(C₁-C₁₀)-alkenyl; and
M is selected from the group consisting of sodium and potassium,
and a physiologically suitable buffer, wherein the composition has an ionic strength of not greater than 0.12, wherein the amount of the compound of formula (I) is 0.001% (w/v) to 0.005% (w/v), and which composition does not contain other antimicrobial agents.

5. The method of claim 4 wherein,
R₁ and R₃ are methyl;
R₂ is selected from the group consisting of (CH₂)₁₁CH₃, (CH₂)₃-NHC(=O)-(CH₂)₁₀CH₃, and (CH₂)₃-NHC(=O)-(CH₂)₁₂CH₃;
R₄ is CH₂CH(Y)CH₂N⁺R₁R₂R₃X⁻, wherein R_{1,} R₂, and R_{3,} are as defined above;
X is chloro;
Y is OH; and
M is sodium.

6. The method of claim 5 wherein the compound of formula (I) is selected from the group consisting of Phospholipid CDM which is a compound of formula (I), wherein:
R₁ and R₃ are methyl;
R₂ is -(CH₂)₁₁CH₃;
R₄ is -CH₂CH(Y)CH₂N⁺R₁R₂R₃X⁻, wherein R₁, R₂, and R₃ are as defined above;
X is chloro;
Y is OH; and
M is sodium,
Phospholipid PTC which is a compound of formula (I), wherein:
R₁ and R₃ are methyl;
R₂ is -(CH₂)₃-NHC(=O)-(CH₂)₁₀CH₃;
R₄ is -CH₂CH(Y)CH₂N⁺R₁R₂R₃X⁻, wherein R₁, R₂, and R₃ are as defined above;
X is chloro;
Y is OH; and
M is sodium,
and Phospholipid PTM which is a compound of formula (I), wherein:
R₁ and R₃ are methyl;
R₂ is -(CH₂)₃-NHC(=O)-(CH₂)₁₂CH₃;
R₄ is CH₂CH(Y)CH₂N⁺R₁R₂R₃X⁻, wherein R₁, R₂, and R₃ are as defined above;
X is chloro;
Y is OH; and
M is sodium.

7. A solution for treating contact lenses, comprising the composition according to claim 1.

8. The solution of claim 7, wherein,
R₁ and R₃ are methyl;
R₂ is selected from the group consisting of (CH₂)₁₁CH₃, (CH₂)₃-NHC(=O)-(CH₂)₁₀CH₃, and (CH₂)₃-NHC(=O)-(CH₂)₁₂CH₃;
R₄ is CH₂CH(Y)CH₂N⁺R₁R₂R₃X⁻, wherein R_{1,} R₂, and R_{3,} are as defined above;
X is chloro;
Y is OH; and
M is sodium.

9. The solution of claim 8 wherein the compound of formula (I) is selected from the group consisting of Phospholipid CDM which is a compound of formula (I), wherein:
R₁ and R₃ are methyl;
R₂ is -(CH₂)₁₁CH₃;
R₄ is -CH₂CH(Y)CH₂N⁺R₁R₂R₃X⁻, wherein R₁, R₂, and R₃ are as defined above;
X is chloro;
Y is OH; and
M is sodium,
Phospholipid PTC which is a compound of formula (I), wherein:
R₁ and R₃ are methyl;
R₂ is -(CH₂)₃-NHC(=O)-(CH₂)₁₀CH₃;
R₄ is -CH₂CH(Y)CH₂N⁺R₁R₂R₃X⁻, wherein R₁, R₂, and R₃ are as defined above;
X is chloro;
Y is OH; and
M is sodium,
and Phospholipid PTM which is a compound of formula (I), wherein:
R₁ and R₃ are methyl;
R₂ is (-(CH₂)₃-NHC(=O)-(CH₂)₁₂CH₃;
R₄ is CH₂CH(Y)CH₂N⁺R₁R₂R₃X⁻, wherein R₁, R₂, and R₃ are as defined above;
X is chloro;
Y is OH; and
M is sodium.

10. A method of disinfecting a contact lens, which comprises contacting the lens with a solution according to claim 7.

11. The method of claim 10 wherein:
R₁ and R₃ are methyl;
R₂ is selected from the group consisting of (CH₂)₁₁CH₃, (CH₂)₃-NHC(=O)-(CH₂)₁₀CH₃, and (CH₂)₃-NHC(=O)-(CH₂)₁₂CH₃;
R₄ is CH₂CH(Y)CH₂N⁺R₁R₂R₃X⁻, wherein R₁, R₂, and R₃, are as defined above;
X is chloro;
Y is OH; and
M is sodium.

12. The method of claim 11 wherein the compound of formula (I) is selected from the group consisting of Phospholipid CDM which is a compound of formula (I), wherein:
R₁ and R₃ are methyl;
R₂ is -(CH₂)₁₁CH₃;
R₄ is -CH₂CH(Y)CH₂N⁺R₁R₂R₃X⁻, wherein R₁, R₂, and R₃ are as defined above;
X is chloro;
Y is OH; and
M is sodium,
Phospholipid PTC which is a compound of formula (I), wherein:
R₁ and R₃ are methyl;
R₂ is -(CH₂)₃-NHC(=O)-(CH₂)₁₀CH₃;
R₄ is -CH₂CH(Y)CH₂N⁺R₁R₂R₃X⁻, wherein R₁, R₂, and R₃ are as defined above;
X is chloro;
Y is OH; and
M is sodium,
and Phospholipid PTM which is a compound of formula (I), wherein:
R₁ and R₃ are methyl;
R₂ is -(CH₂)₃-NHC(=O)-(CH₂)₁₂CH₃;
R₄ is CH₂CH(Y)CH₂N⁺R₁R₂R₃X⁻, wherein R₁, R₂, and R₃ are as defined above;
X is chloro;
Y is OH; and
M is sodium.

13. The composition of claim 1, wherein the composition is ophthalmic, otic or nasal.

## Patentansprüche

1. Sterile pharmazeutische Zusammensetzung, umfassend eine konservierend wirkende Menge einer Verbindung der folgenden Formel 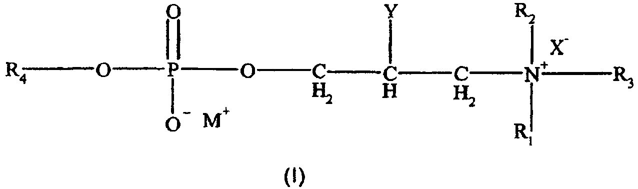 wobei:
R₁ und R₃ (C₁-C₆)-Alkyl sind;
R₂ ausgewählt ist aus der Gruppe, bestehend aus Wasserstoff und (C₁-C₁₆)-Alkyl, das gegebenenfalls substituiert ist durch NHC(=O)-(CH₂)₁₀CH₃ oder
NHC(=O)-(CH₂)₁₂CH₃;
R₄ ausgewählt ist aus der Gruppe, bestehend aus Wasserstoff und
CH₂CH(Y)CH₂N⁺R₁R₂R₃X⁻, wobei R₁, R₂ und R₃ wie oben definiert sind;
X Halogen ist;
Y ausgewählt ist aus der Gruppe, bestehend aus OH, O-(C₁-C₁₀)-Alkyl und
O-(C₁-C₁₀)-Alkenyl;
M ausgewählt ist aus der Gruppe, bestehend aus Natrium und Kalium;
einen physiologisch geeigneten Puffer und ein pharmazeutisch akzeptables Vehikel dafür, wobei die Zusammensetzung eine Ionenstärke von nicht mehr als 0,12 aufweist, wobei die Menge der Verbindung der Formel (I) 0,001 % (Gew./Vol.) bis 0,005 % (Gew./Vol.) beträgt, und wobei die Zusammensetzung keine anderen antimikrobiellen Mittel enthält.

2. Zusammensetzung nach Anspruch 1, wobei:
R₁ und R₃ Methyl sind;
R₂ ausgewählt ist aus der Gruppe, bestehend aus (CH₂)₁₁CH₃,
(CH₂)₃-NHC(=O)-(CH₂)₁₀CH₃ und (CH₂)₃-NHC(=O)-(CH₂)₁₂CH₃;
R₄ CH₂CH(Y)CH₂N⁺R₁R₂R₃X⁻ ist, wobei R₁, R₂ und R₃ wie oben definiert sind;
X Chlor ist;
Y OH ist und
M Natrium ist.

3. Zusammensetzung nach Anspruch 2, wobei die Verbindung der Formel (I) ausgewählt ist aus der Gruppe, bestehend aus Phospholipid CDM, das eine Verbindung der Formel (I) ist,
wobei:
R₁ und R₃ Methyl sind;
R₂ -(CH₂)₁₁CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
Phospholipid PTC, das eine Verbindung der Formel (I) ist, wobei:
R₁ und R₃ Methyl sind;
R₂ -(CH₂)₃-NHC(=O)-(CH₂)₁₀CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
und Phospholipid PTM, das eine Verbindung der Formel (I) ist, wobei:
R₁ und R₃ Methyl sind;
R₂ -(CH₂)₃-NHC(=O)-(CH₂)₁₂CH₃ ist;
R₄ CH₂CH(Y)CH₂N⁺R,R₂R₃X⁻ ist, wobei R₁, R₂ und R₃ wie oben definiert sind;
X Chlor ist;
Y OH ist und
M Natrium ist.

4. Verfahren zum Konservieren einer pharmazeutischen Zusammensetzung vor mikrobieller Kontamination, das das Zugeben zu der Zusammensetzung einer konservierend wirkenden Menge einer Verbindung der folgenden Formel 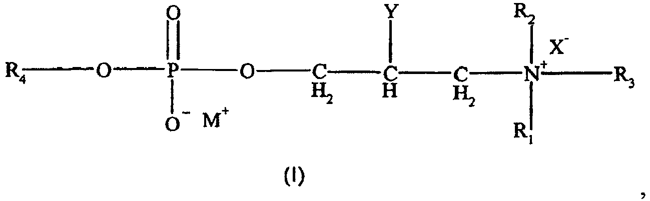 wobei:
R₁ und R₃ (C₁-C₆)-Alkyl sind;
R₂ ausgewählt ist aus der Gruppe, bestehend aus Wasserstoff und (C₁-C₁₆)-Alkyl, das gegebenenfalls substituiert ist durch NHC(=O)-(CH₂)₁₀CH₃ oder
NHC(=O)-(CH₂)₁₂CH₃;
R₄ ausgewählt ist aus der Gruppe, bestehend aus Wasserstoff und
CH₂CH(Y)CH₂N⁺R₁R₂R₃X⁻, wobei R₁, R₂ und R₃ wie oben definiert sind;
X Halogen ist;
Y ausgewählt ist aus der Gruppe, bestehend aus OH, O-(C₁-C₁₀)-Alkyl und
O-(C₁-C₁₀)-Alkenyl; und
M ausgewählt ist aus der Gruppe, bestehend aus Natrium und Kalium,
und eines physiologisch geeigneten Puffers umfasst, wobei die Zusammensetzung eine Ionenstärke von nicht mehr als 0,12 hat, wobei die Menge der Verbindung der Formel (I) 0,001 % (Gew./Vol.) bis 0,005 % (Gew./Vol.) beträgt, und wobei die Zusammensetzung keine anderen antimikrobiellen Mittel enthält.

5. Verfahren nach Anspruch 4, wobei
R₁ und R₃ Methyl sind;
R₂ ausgewählt ist aus der Gruppe, bestehend aus (CH₂)₁₁CH₃, (CH₂)₃-NHC(=O)-(CH₂)₁₀CH₃ und (CH₂)₃-NHC(=O)-(CH₂)₁₂CH₃;
R₄ CH₂CH(Y)CH₂N⁺R₁R₂R₃X⁻ ist, wobei R₁, R₂ und R₃ wie oben definiert sind;
X Chlor ist;
Y OH ist und
M Natrium ist.

6. Verfahren nach Anspruch 5, wobei die Verbindung der Formel (I) ausgewählt ist aus der Gruppe, bestehend aus Phospholipid CDM, das eine Verbindung der Formel (I) ist, wobei:
R₁ und R₃ Methyl sind;
R₂-(CH₂)₁₁CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
Phospholipid PTC, das eine Verbindung der Formel (I) ist, wobei:
R₁ und R₃ Methyl sind;
R₂ -(CH₂)₃-NHC(=O)-(CH₂)₁₀CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
und Phospholipid PTM, das eine Verbindung der Formel (I) ist, wobei:
R₁ und R₃ Methyl sind;
R₂ -(CH₂)₃-NHC(=O)-(CH₂)₁₂CH₃ ist;
R₄ CH₂CH(Y)CH₂N⁺R₁R₂R₃X⁻ ist, wobei R₁, R₂ und R₃ wie oben definiert sind;
X Chlor ist;
Y OH ist und
M Natrium ist.

7. Lösung zur Behandlung von Kontaktlinsen, umfassend die Zusammensetzung nach Anspruch 1.

8. Lösung nach Anspruch 7, wobei
R₁ und R₃ Methyl sind;
R₂ ausgewählt ist aus der Gruppe, bestehend aus (CH₂)₁₁CH₃, (CH₂)₃-NHC(=O)-(CH₂)₁₀CH₃ und (CH₂)₃-NHC(=O)-(CH₂)₁₂CH₃;
R₄ CH₂CH(Y)CH₂N⁺R₁R₂R₃X⁻ ist, wobei R₁, R₂ und R₃ wie oben definiert sind;
X Chlor ist;
Y OH ist und
M Natrium ist.

9. Lösung nach Anspruch 8, wobei die Verbindung der Formel (I) ausgewählt ist aus der Gruppe, bestehend aus Phospholipid CDM, das eine Verbindung der Formel (I) ist, wobei:
R₁ und R₃ Methyl sind;
R₂ -(CH₂)₁₁CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
Phospholipid PTC, das eine Verbindung der Formel (I) ist, wobei:
R₁ und R₃ Methyl sind;
R₂ -(CH₂)₃-NHC(=O)-(CH₂)₁₀CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
und Phospholipid PTM, das eine Verbindung der Formel (I) ist, wobei:
R₁ und R₃ Methyl sind;
R₂ -(CH₂)₃-NHC(=O)-(CH₂)₁₂CH₃ ist;
R₄ CH₂CH(Y)CH₂N⁺R₁R₂R₃X ist, wobei R₁, R₂ und R₃ wie oben definiert sind;
X Chlor ist;
Y OH ist und
M Natrium ist.

10. Verfahren zum Desinfizieren einer Kontaktlinse, umfassend das Kontaktieren der Linse mit einer Lösung nach Anspruch 7.

11. Verfahren nach Anspruch 10, wobei
R₁ und R₃ Methyl sind;
R₂ ausgewählt ist aus der Gruppe, bestehend aus (CH₂)₁₁CH₃, (CH₂)₃-NHC(=O)-(CH₂)₁₀CH₃ und (CH₂)₃-NHC(=O)-(CH₂)₁₂CH₃;
R₄ CH₂CH(Y)CH₂N⁺R₁R₂R₃X⁻ ist, wobei R₁, R₂ und R₃ wie oben definiert sind;
X Chlor ist;
Y OH ist und
M Natrium ist.

12. Verfahren nach Anspruch 11, wobei die Verbindung der Formel (I) ausgewählt ist aus der Gruppe, bestehend aus Phospholipid CDM, das eine Verbindung der Formel (I) ist, wobei:
R₁ und R₃ Methyl sind;
R₂ -(CH₂)₁₁CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
Phospholipid PTC, das eine Verbindung der Formel (I) ist, wobei:
R₁ und R₃ Methyl sind;
R₂ -(CH₂)₃-NHC(=O)-(CH₂)₁₀CH₃ ist;
R₄ -CH₂CH(Y)CH₂N⁺R₁R₂R₃X⁻ ist, wobei R₁, R₂ und R₃ wie oben definiert sind;
X Chlor ist;
Y OH ist und
M Natrium ist,
und Phospholipid PTM, das eine Verbindung der Formel (I) ist, wobei:
R₁ und R₃ Methyl sind;
R₂ -(CH₂)₃-NHC(=O)-(CH₂)₁₂CH₃ ist;
R₄ CH₂CH(Y)CH₂N⁺R₁R₂R₃X⁻ ist, wobei R₁, R₂ und R₃ wie oben definiert sind;
X Chlor ist;
Y OH ist und
M Natrium ist.

13. Zusammensetzung nach Anspruch 1, wobei die Zusammensetzung für das Auge, das Ohr oder die Nase ist.

## Revendications

1. Composition pharmaceutique stérile comprenant une quantité préservatrice efficace d'un composé de la formule suivante, 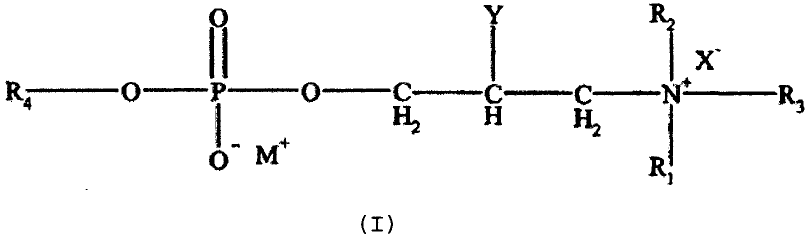 dans laquelle :
R₁ et R₃ représentent un alkyle-(C₁-C₆) ;
R₂ est choisi dans le groupe consistant en un hydrogène et un alkyle-(C₁-C₁₆) éventuellement substitué par NHC(=O)-(CH₂)₁₀CH₃ ou NHC(=O)-(CH₂)₁₂CH₃ ;
R₄ est choisi dans le groupe consistant en un hydrogène et CH₂CH(Y)CH₂N⁺R₁R₂R₃X⁻ dans laquelle R₁, R₂, et R₃, sont tels que définis ci-dessus ;
X représente un halogéno ;
Y est choisi dans le groupe consistant en OH, O-alkyle-(C₁-C₁₀) et O-alcényle-(C₁-C₁₀) ;
M est choisi dans le groupe consistant en du sodium et du potassium ;
un tampon physiologiquement approprié, et un véhicule pharmaceutiquement acceptable associé, ladite composition ayant une force ionique inférieure ou égale à 0,12, dans laquelle la quantité du composé de formule (I) est située dans la plage allant de 0,001 % (p/v) à 0,005 % (p/v), et ladite composition ne contenant pas d'autres agents antimicrobiens.

2. Composition selon la revendication 1, dans laquelle :
R₁ et R₃ représentent un méthyle ;
R₂ est choisi dans le groupe consistant en (CH₂)₁₁CH₃, (CH₂)₃-NHC(=O)-(CH₂)₁₀CH₃, et
(CH₂)₃-NHC(=O)-(CH₂)₁₂CH₃ ;
R₄ représente CH₂CH(Y)CH₂N⁺R₁R₂R₃X⁻, dans laquelle R₁, R₂,
et R₃, sont tels que définis ci-dessus ;
X représente un chloro ;
Y représente OH ; et
M représente un sodium.

3. Composition selon la revendication 2, dans laquelle le composé de formule (I) est choisi dans le groupe consistant en un phospholipide CDM qui est un composé de formule (I), dans laquelle :
R₁ et R₃ représentent un méthyle ;
R₂ représente -(CH₂)₁₁CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
un phospholipide PTC qui est un composé de formule (I),
dans laquelle :
R₁ et R₃ représentent un méthyle ;
R₂ représente -(CH₂)₃-NHC(=O)-(CH₂)₁₀CH3 ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
et un phospholipide PTM qui est un composé de formule (I), dans laquelle :
R₁ et R₃ représentent un méthyle ;
R₂ représente -(CH₂)₃-NHC(=O)-(CH₂)₁₂CH_{3 ;}
R₄ représente CH₂CH(Y)CH₂N⁺R₁R₂R₃X⁻, dans laquelle R₁, R₂,
et R₃ sont tels que définis ci-dessus ;
X représente un chloro ;
Y représente OH ; et
M représente un sodium.

4. Procédé permettant de préserver une composition pharmaceutique d'une contamination microbienne qui consiste à ajouter à la composition une quantité préservatrice efficace d'un composé de la formule suivante, 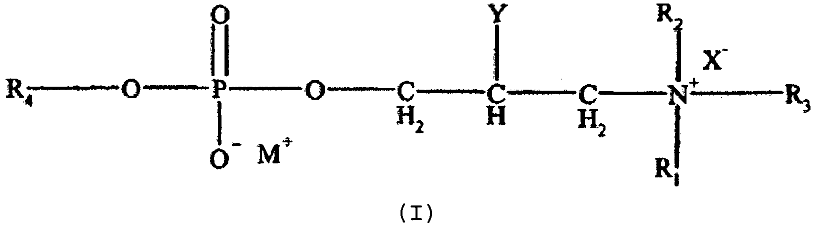 dans laquelle :
R₁ et R₃ représentent un alkyle-(C₁-C₆) ;
R₂ est choisi dans le groupe consistant en un hydrogène et un alkyle-(C₁-C₁₆) éventuellement substitué par NHC(=O)-(CH₂)₁₀CH₃ ou NHC(=O)-(CH₂)₁₂CH₃ ;
R₄ est choisi dans le groupe consistant en un hydrogène et CH₂CH(Y)CH₂N⁺R₁R₂R₃X dans laquelle R₁, R₂, et R₃ sont tels que définis ci-dessus ;
X représente un halogéno ;
Y est choisi dans le groupe consistant en OH, O-alkyle-(C₁-C₁₀) et O-alcényle-(C₁-C₁₀) ; et
M est choisi dans le groupe consistant en du sodium et du potassium, et un tampon physiologiquement approprié, ladite composition ayant une force ionique inférieure ou égale à 0,12, dans laquelle la quantité du composé de formule (I) est située dans la plage allant de 0,001 % (p/v) à 0,005 % (p/v), et ladite composition ne contenant pas d'autres agents antimicrobiens.

5. Procédé selon la revendication 4, dans lequel,
R₁ et R₃ représentent un méthyle ;
R₂ est choisi dans le groupe consistant en (CH₂)₁₁CH₃, (CH₂)₃-NHC(=O)-(CH₂)₁₀CH₃, et (CH₂)₃-NHC(=O)-(CH₂)₁₂CH₃ ;
R₄ représente CH₂CH(Y)CH₂N⁺R₁R₂R₃X⁻, dans laquelle R₁, R₂, et R₃, sont tels que définis ci-dessus ;
X représente un chloro ;
Y représente OH ; et
M représente un sodium.

6. Procédé selon la revendication 5, dans laquelle le composé de formule (I) est choisi dans le groupe consistant en un phospholipide CDM qui est un composé de formule (I), dans laquelle :
R₁ et R₃ représentent un méthyle ;
R₂ représente -(CH₂)₁₁CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
un phospholipide PTC qui est un composé de formule (I), dans laquelle :
R₁ et R₃ représentent un méthyle ;
R₂ représente -(CH₂)₃-NHC(=O)-(CH₂)₁₀CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
et un phospholipide PTM qui est un composé de formule (I), dans laquelle :
R₁ et R₃ représentent un méthyle ;
R₂ représente -(CH₂)₃-NHC(=O)-(CH₂)₁₂CH₃ ;
R₄ représente CH₂CH(Y)CH₂N⁺R₁R₂R₃X⁻, dans laquelle R₁, R₂,
et R₃ sont tels que définis ci-dessus ;
X représente un chloro ;
Y représente OH ; et
M représente un sodium.

7. Solution de traitement de lentilles de contact, comprenant la composition selon la revendication 1.

8. Solution selon la revendication 7, dans laquelle,
R₁ et R₃ représentent un méthyle ;
R₂ est choisi dans le groupe consistant en (CH₂)₁₁CH₃, (CH₂)₃-NHC(=O)-(CH₂)₁₀CH₃, et (CH₂)₃-NHC(=O)-(CH₂)₁₂CH₃ ;
R₄ représente CH₂CH(Y)CH₂N⁺R₁R₂R₃X⁻, dans laquelle R₁, R₂, et R₃, sont tels que définis ci-dessus ;
X représente un chloro ;
Y représente OH ; et
M représente un sodium.

9. Solution selon la revendication 8, dans laquelle le composé de formule (I) est choisi dans le groupe consistant en un phospholipide CDM qui est un composé de formule (I), dans laquelle :
R₁ et R₃ représentent un méthyle ;
R₂ représente -(CH₂)₁₁CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
un phospholipide PTC qui est un composé de formule (I), dans laquelle :
R₁ et R₃ représentent un méthyle ;
R₂ représente -(CH₂)₃-NHC(=O)-(CH₂)₁₀CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
et un phospholipide PTM qui est un composé de formule (I), dans laquelle :
R₁ et R₃ représentent un méthyle ;
R₂ représente (-(CH₂)₃-NHC(=O)-(CH₂)₁₂CH₃ ;
R₄ représente CH₂CH(Y)CH₂N⁺R₁R₂R₃X⁻, dans laquelle R₁, R₂,
et R₃ sont tels que définis ci-dessus ;
X représente un chloro ;
Y représente OH ; et
M représente un sodium.

10. Procédé de désinfection d'une lentille de contact, qui comprend la mise en contact de la lentille de contact avec une solution selon la revendication 7.

11. Procédé selon la revendication 10, dans lequel :
R₁ et R₃ représentent un méthyle ;
R₂ est choisi dans le groupe consistant en (CH₂)₁₁CH₃, (CH₂)₃-NHC(=O)-(CH₂)₁₀CH₃, et (CH₂)₃-NHC(=O)-(CH₂)₁₂CH₃ ;
R₄ représente CH₂CH(Y)CH₂N⁺R₁R₂R₃X⁻, dans laquelle R₁, R₂,
et R₃, sont tels que définis ci-dessus ;
X représente un chloro ;
Y représente OH ; et
M représente un sodium.

12. Procédé selon la revendication 11, dans lequel le composé de formule (I) est choisi dans le groupe consistant en un phospholipide CDM qui est un composé de formule (I), dans laquelle :
R₁ et R₃ représentent un méthyle ;
R₂ représente -(CH₂)₁₁CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
un phospholipide PTC qui est un composé de formule (I), dans laquelle :
R₁ et R₃ représentent un méthyle ;
R₂ représente -(CH₂)₃-NHC(=O)-(CH₂)₁₀CH₃ ;
R₄ représente -CH₂CH(Y)CH₂N⁺R₁R₂R₃X⁻, dans laquelle R₁, R₂, et R₃ sont tels que définis ci-dessus ;
X représente un chloro ;
Y représente OH ; et
M représente un sodium,
et un phospholipide PTM qui est un composé de formule (I), dans laquelle :
R₁ et R₃ représentent un méthyle ;
R₂ représente -(CH₂)₃-NHC(=O)-(CH₂)₁₂CH₃ ;
R₄ représente CH₂CH(Y)CH₂N⁺R₁R₂R₃X⁻, dans laquelle R₁, R₂,
et R₃ sont tels que définis ci-dessus ;
X représente un chloro ;
Y représente OH ; et
M représente un sodium.

13. Composition selon la revendication 1, dans laquelle la composition est une composition ophtalmique, otique ou nasale.
